# EUROPEAN PATENT APPLICATION

(11) **EP 4 467 071 A1**
(43) Date of publication of application: **27.11.2024**
(21) Application number: 24177055.1
(22) Date of filing: 21.05.2024
(51) Int. Cl.: A61B 5/024, A61B 5/1455, A61B 5/00

(54) **A MOISTURE PROTECTED PHOTOPLETHYSMOGRAPHIC LASER HOUSING**

(30) Priority: 22.05.2023 US 202318321195
(71) Applicant: Zynex Monitoring Solutions, Inc., Englewood, CO 80112 (US)
(72) Inventor: Pologe, Jonas Alexander, Colorado, 80305 (US); Delianides, Theodore Philip, Colorado, 80305 (US)
(74) Representative: Forresters IP LLP

(57) **Abstract**

A photoplethysmographic device includes a photoplethysmographic monitor, a laser housing electronically coupled to the photoplethysmographic monitor, at least two diode lasers, which generate laser light, positioned within the laser housing, the photoplethysmographic monitor providing electronic control of the at least two diode lasers, a desiccant fully enclosed within the laser housing, the desiccant positioned within the laser housing such that the desiccant does not make physical contact with the at least two diode lasers and/or interfere with the laser light, and a sensor in communication with the photoplethysmographic monitor and adapted to output the laser light from the at least two diode lasers. The sensor includes a photodetector, and the photoplethysmographic monitor receives and processes photoplethysmographic data from the sensor.

## Description

### FIELD OF THE INVENTION

This invention is in the field of photoplethysmography.

### BACKGROUND OF THE INVENTION

In the science of photoplethysmography, light is used to illuminate or trans-illuminate living tissue for the purpose of providing noninvasive measurements of blood analytes, hemodynamic parameters, and/or tissue properties. In this monitoring modality light is directed into living tissue (the "tissue-under-test") and a portion of the light that is not absorbed by the tissue-under-test, or scattered in some other direction, is detected a short distance from the point at which the light entered the tissue. The detected incoming light, or photoplethysmographic signal (the pulsatile optical signal exiting the living tissue), is converted into an electronic signal (or photoplethysmographic data) that is used to calculate blood analyte levels such as, but not limited to, arterial blood oxygen saturation, total hemoglobin concentration, serum bilirubin levels, and/or hemodynamic parameters such as heart rate, cardiac output, blood oxygen content, and/or tissue perfusion. A device which detects and processes photoplethysmographic data and/or signals, to measure the levels of various blood analytes and/or various hemodynamic parameters is a photoplethysmographic measurement apparatus, photoplethysmographic device, or photoplethysmographic instrument. A photoplethysmographic device typically includes a photoplethysmographic monitor combined with a sensor. The first widespread commercially-used photoplethysmographic device in medicine was the pulse oximeter, a photoplethysmographic device designed to measure arterial blood oxygen saturation.

The photoplethysmographic monitor includes electronic circuitry for controlling light emitters that emit light which is then incident on the tissue-under-test. These electronics may also perform the functions of receiving and processing the photoplethysmographic signals emitted from the tissue-under-test, converting these photoplethysmographic data into the measurements (blood analyte measurements and/or hemodynamic parameter measurements and/or other measurements of the tissue-under-test) to be displayed, and displaying these measurements on some form of user display, such as an LCD display. Photoplethysmographic devices come in many forms including, but not limited to: standalone monitors having a built-in display and detachable sensors, or electronics modules that transmit the measurements for display by a remote display, on an attached computer, or by another display device such as a smart phone. Photoplethysmographic devices may also be fully integrated devices where the monitor, display, and sensor are integrated into a single device, as is seen in fingertip pulse oximeters. The photoplethysmographic monitor may also provide other functions and include other components such as a keypad, buttons, or touchscreen for user input, visible indicators (e.g. LED lights), audible enunciators (e.g. speakers) for alarms, and/or wired or wireless connection ports (e.g. USB, RS232, Ethernet) for digital and analog inputs and outputs.

The sensor typically includes at least an emitter and a photodetector. The sensor positions these elements so that light emitted from the emitter is incident on the tissue-under-test and a portion of that light, having passed through living tissue and having been modulated by the pulsatile variation in blood volume in the tissue-under-test, can be received by the photodetector. In conventional LED-based pulse oximetry, the emitter includes at least two LEDs, one with its spectral content typically centered around 660 nanometers (nm) and one with its spectral content typically centered in the near infrared at about 900nm or 940nm. Herein, the term emitter is used to refer to the light from all light sources that is made incident on the tissue-under-test for photoplethysmographic measurement. These light sources may include light from LEDs, lasers, a tungsten light, or any combination of light sources used for photoplethysmographic measurement.

The sensor arrangement most commonly used in photoplethysmography over the last 40 years has been a fingertip sensor that positions light emitting diodes (LEDs) and a photodetector on opposite sides of the tissue-under-test, typically a finger or an ear. This arrangement was predated by the Minolta-Marquest SM-32 Oxygen Saturation Monitor which employed, as its emitter, a tungsten light source physically housed in the photoplethysmographic monitor and employed fiber optic bundles for delivery of light to and from the tissue-under-test. The advent of inexpensive and efficient LED emitters in photoplethysmography in the 1980s resulted in a more effective device that allowed efficient light delivery to the tissue-under-test, and this has become the preferred arrangement in present-day pulse oximeters. In certain circumstances, however, for example oximetry sensors used in the high magnetic field environment of MRI (Magnetic Resonance Imaging) devices, there is still a need for fiber optic delivery of the light to and from the tissue-under-test. In such cases, the LEDs may be located within the photoplethysmographic monitor, or even partway along a patient cable connecting the photoplethysmographic monitor to the sensor, and the light out of the LEDs is directed into one or more optical fibers (or light guides). The optical fibers are routed to the sensor where the light emitted by the LEDs is then output by the sensor and incident on the tissue-under-test, with a second set of optical fibers used to return light to a photodetector located outside of the MRI environment.

A more recent improvement to the field of photoplethysmography has been the introduction of laser light sources to replace, or use in combination with, one or more LEDs. The introduction of lasers in pulse oximetry provides the opportunity to expand the measurement capabilities of photoplethysmography from the measurement of one blood analyte, specifically arterial oxygen saturation, to the measurement of multiple blood analytes and physiological parameters. The narrow spectral bandwidth of laser light improves the resolution, accuracy, and precision of photoplethysmographic measurements, thus making accurate measurement of analytes such as oxyhemoglobin, carboxyhemoglobin, methemoglobin, reduced hemoglobin, and a number of other analytes, technically feasible.

Not unexpectedly, the use of lasers in photoplethysmography introduces a number of new problems to the design and implementation of commercially-viable photoplethysmographic devices. Among these is the problem of how to create a laser housing for a photoplethysmographic device that contains one or more laser light sources, each laser potentially coupled to an optical fiber, where the laser light sources are stable and reliable over time, and where the design of the housing allows for efficient and low-cost manufacturing processes. It is the solution to this problem that the current invention is intended to address.

### SUMMARY OF THE INVENTION

The present invention overcomes the problems and disadvantages associated with current strategies and designs and provides new systems and methods of manufacturing photoplethysmographic devices.

In accordance with one embodiment, a photoplethysmographic device, comprising: a photoplethysmographic monitor; a laser housing, or a set of laser housings, electronically coupled to the photoplethysmographic monitor; at least two diode lasers, which generate laser light, positioned within the one or more laser housings; the photoplethysmographic monitor providing electronic control of the at least two diode lasers; a desiccant fully enclosed within the one or more laser housings; the desiccant positioned within the laser housing such that the desiccant does not make physical contact with the at least two diode lasers and/or interfere with the laser light; and a sensor in communication with the photoplethysmographic monitor and adapted to output the laser light from the at least two diode lasers; the sensor comprising a photodetector, wherein the photoplethysmographic monitor receives and processes photoplethysmographic data from the sensor.

One embodiment of the invention is directed to a photoplethysmographic device. The device comprises a photoplethysmographic monitor, a laser housing electronically coupled to the photoplethysmographic monitor, at least two diode lasers, which generate laser light, positioned within the laser housing, the photoplethysmographic monitor providing electronic control of the at least two diode lasers, a desiccant fully enclosed within the laser housing, and a sensor in communication with the photoplethysmographic monitor and adapted to output the laser light from the at least two diode lasers. The desiccant is positioned within the laser housing such that the desiccant does not make physical contact with the at least two diode lasers and/or interfere with the laser light. The sensor comprises a photodetector, wherein the photoplethysmographic monitor receives and processes photoplethysmographic data from the sensor.

Preferably, the desiccant is held in position within the laser housing by an adhesive. The adhesive is preferably at least one of an epoxy, a silicone elastomer, a cyanoacrylate, a hot melt glue, and combinations thereof. In a preferred embodiment, the desiccant is isolated from the at least two diode lasers by a physical separator positioned within the laser housing, wherein the physical separator allows air movement between the desiccant and the at least two diode lasers.

Preferably, the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity.

In a preferred embodiment, the laser housing further comprises at least one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor.

Preferably, the laser housing is non-hermetic. The laser housing preferably includes at least one aluminum part. In a preferred embodiment, the desiccant is at least one of silica gel, clay, activated charcoal, calcium sulfate, calcium chloride, calcium oxide, molecular sieve material, zeolite, and combinations thereof. Preferably, the desiccant drops the relative humidity within the laser housing to less than 3.5% at 25°C, 760mm of mercury pressure.

Another embodiment of the invention is directed to a photoplethysmographic device. The devices comprises a photoplethysmographic monitor, at least two laser housings electronically coupled to the photoplethysmographic monitor, at least one diode laser, which generates laser light, positioned within each of the at least two laser housings, a desiccant fully enclosed within each of the at least two laser housings, and a sensor in communication with the photoplethysmographic monitor and adapted to output the laser light from the diode lasers. Thee photoplethysmographic monitor provides electronic control of each of the diode lasers. Each desiccant is positioned within each of the at least two laser housings such that each desiccant does not make physical contact with the diode lasers and/or interfere with the laser light. The sensor comprises a photodetector, wherein the photoplethysmographic monitor receives and processes photoplethysmographic data from the sensor.

In a preferred embodiment, the desiccant is held in position within the laser housing by an adhesive. Preferably, the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity.

Preferably, each laser housing further comprises one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor. Each laser housing is preferably non-hermetic. In a preferred embodiment, the desiccant is at least one of silica gel, clay, activated charcoal, calcium sulfate, calcium chloride, calcium oxide, molecular sieve material, zeolite, and combinations thereof. Preferably, the desiccant drops the relative humidity within the laser housing to less than 3.5% at 25°C, 760mm of mercury pressure.

Another embodiment of the invention is directed to a method of manufacturing a photoplethysmographic device. The method comprises the steps of providing a photoplethysmographic monitor, providing at least two diode lasers that each generate laser light,
positioning each diode laser within a laser housing, wherein one or more diode lasers are contained within one or more laser housings, electronically coupling each diode laser to the photoplethysmographic monitor, positioning a desiccant within each of the one or more laser housings, and coupling a sensor to the photoplethysmographic monitor, wherein the desiccant is positioned such that the desiccant does not interfere with the laser light. The sensor is in communication with the photoplethysmographic monitor and is adapted to guide the laser light from the diode lasers into a tissue-under-test. The photoplethysmographic monitor receives and processes photoplethysmographic data from the sensor.

In a preferred embodiment, the step of positioning the desiccant within each laser housing is by an adhesive. Preferably, the adhesive is at least one of an epoxy, a silicone elastomer, a cyanoacrylate, a hot melt glue, and combinations thereof. In a preferred embodiment, the desiccant is selected to adsorb less than 25% of its maximum water absorption capacity when exposed for less than 5 minutes to ambient air at standard temperature and pressure and no more than 75% relative humidity, whereby the desiccant can be sealed within the laser housing in ambient air while still maintaining at least 75% of the desiccant's water absorption capacity.

The method preferably further comprises coupling one optical fiber to each diode laser, the optical fiber transiting the associated housing and transmitting the laser light emitted by the diode laser to the sensor directly or through two or more serially-connected optical fibers or other light pipes. Preferably, the sensor comprises a laser light output aperture and a photodetector and wherein the sensor is capable of transmitting photoplethysmographic data from the photodetector to the photoplethysmographic monitor. At least one of the one or more laser housings is preferably non-hermetic.

Preferably, the desiccant is at least one of silica gel, clay, activated charcoal, calcium sulfate, calcium chloride, calcium oxide, molecular sieve material, zeolite, and combinations thereof. In a preferred embodiment, the desiccant reduces the relative humidity within the at least one or more laser housings to less than 3.5% at 25°C, 760mm of mercury pressure.

Included in the advantages of this invention are the ability to use a non-hermetic laser housing(s), manufactured from inexpensive materials, without the need to bake out the housing before sealing, while still protecting the diode laser(s) contained within the housing(s) from residual moisture contained within the housing or moisture migrating into the housing after it is sealed. This and other advantages will become apparent from review of the following description and the accompanying drawings.

Other embodiments and advantages of the invention are set forth in part in the description, which follows, and in part, may be obvious from this description, or may be learned from the practice of the invention.

### BRIEF DESCRIPTION OF THE DRAWINGS

The foregoing features of embodiments will be more readily understood by reference to the following detailed description, taken with reference to the accompanying drawings, in which:
- *FIG. 1*:: Photoplethysmographic device.
- *FIG. 2*:: Laser housing containing a single diode laser and desiccant.
- *FIG. 3*:: Laser housing containing two or more diode lasers and desiccant.
- *FIG. 4*:: Laser housing containing two or more diode lasers, desiccant, and a physical separator.

### DETAILED DESCRIPTION OF THE INVENTION

As embodied and broadly described herein, the disclosures herein provide detailed embodiments of the invention. However, the disclosed embodiments are merely exemplary of the invention that may be embodied in various and alternative forms. Therefore, there is no intent that specific structural and functional details should be limiting, but rather the intention is that they provide a basis for the claims and as a representative basis for teaching one skilled in the art to variously employ the present invention.

In developing a laser housing for a photoplethysmographic device, it was discovered that one of the key causes for a lack of long-term stability and reliability is the presence or introduction of water or water vapor inside the laser housing. One potential solution appeared to be to seal the laser housing in a dry nitrogen environment. However, it was discovered that there may be moisture hidden within, or bound to, the internal surfaces of the laser housing, particularly in a metallic (e.g. aluminum) housing, that would become free moisture inside the enclosed space of the laser housing once it was sealed, even if the housing was fully hermetic and was hermetically sealed in a dry nitrogen atmosphere. Additionally, trying to perform the seal in a dry nitrogen atmosphere was a clumsy labor- and equipment-intensive process that was not conducive to low-cost manufacturing.

Further, even if the atmosphere internal to the laser housing is totally free of moisture upon sealing, with the exception of a few specific fully-hermetic materials, notably glass, ceramic, and metal, most, if not all, other packages will still have a non-zero water vapor transfer rate, resulting in diffusion of water vapor from the ambient air into the laser housing over time. To compound the problem, a laser housing package for a photoplethysmographic device must allow for electrical connections and potentially optical fibers to pass through the laser housing, creating the potential for additional routes of entry for water vapor.

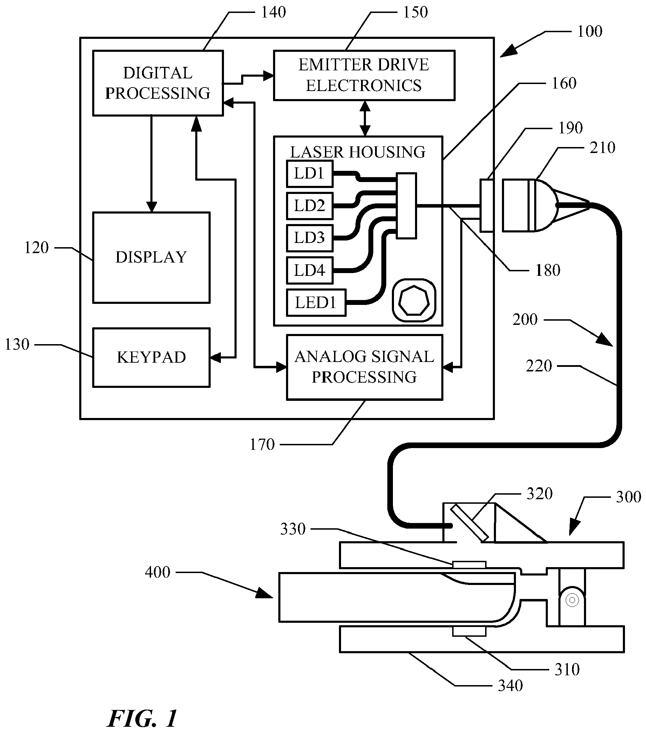

One embodiment of a photoplethysmographic device, as shown in FIG. 1, comprises a photoplethysmographic monitor **100** a patient cable **200,** and a sensor **300.** The photoplethysmographic monitor or electronics module **100** processes photoplethysmographic signals. The monitor **100** may also include a visual display **120** which presents, or outputs, information such as, but not limited to, measured values for the blood analytes, hemodynamic parameters, other tissue properties such as tissue perfusion levels, waveforms, alarms, and device status information to the clinician or end user of the photoplethysmographic device. The monitor **100** also may include a user interface or control panel such as a keypad **130,** or a touchscreen integral to the display, for controlling the operation of the device. The monitor **100** further provides digital processor **140** and analog signal processor **170.** Digital processing preferably includes handling communication to the display **120,** processing inputs from the user interface **130,** as well as controlling the timing of the emitter drive electronics **150** and any timing required in the analog signal processor **170,** and controlling or routing output values generated by the analog signal processor **170.** Additionally, the emitter drive electronics **150** may be electronically coupled to the laser housing **160** to control the diode lasers, also referred to as laser diodes turning them on and off sequentially, in a time-division-multiplexed manner, so that one laser at a time is turned on and so that the monitor **100** knows what diode laser is associated with analog signal returned from the sensor **300.** While one laser housing 160 is shown, there may be multiple laser housings. For example, each diode laser may be housed in a single laser housing or multiple diode lasers may share a laser housing. The emitter drive electronics **150** may also control, or receive data from, other electrical or optoelectronic elements contained within the laser housing **160.** The photoplethysmographic device may also have a power source, which could comprise a battery, wall current, or another source of power, and a power conditioning device.

The patient cable **200** preferably connects the photoplethysmographic monitor **100** to the sensor **300.** Patient cable connector **210** couples cable **220** to the monitor **100** at the monitor sensor connector **190.** The cable **220** transmits, or communicates, the light generated in the laser housing(s) **160** from the monitor **100** to the sensor **300** and returns the photoplethysmographic data or signals received from the tissue-under-test **400** to the monitor **100** for analog and digital signal processing and/or for the measured or calculated parameters to be output by display **120.**

In this embodiment, the sensor **300** receives the diode laser light signals transmitted through the patient cable **200.** Mirror **320** reflects the diode laser light signals through the output aperture **330** into the tissue-under-test **400.** A portion of the light incident on the tissue-under-test is emitted from the tissue and incidents on the detector **310** in the sensor **300.** This light signal emitted from the tissue-under-test has been modulated, in intensity, by the pulsation of the blood in tissue-under-test **400,** creating an output or emitted light signal with a pulsatile component, also referred to as a photoplethysmographic signal.

The photoplethysmographic signal could also be received optically by, for example, an optical fiber and transmitted or communicated back to the monitor **100** via an optical fiber within the patient cable **200** where it could then be converted by a detector, which may be a photodiode or another type of photodetector, into an electronic signal for processing.

The light incident on the tissue-under-test **400** is, in viable tissue, modulated by the pulsation of blood into and out of the tissue. As the blood volume in the tissue-under-test **400** increases the optical density of the tissue increases and the light level emitted or output by the tissue and incident on the detector **310** decreases. Conversely as the blood volume in the tissue-under-test **400** decreases the optical density of the tissue decreases and the light level emitted by the tissue and incident on the detector **310** increases. This fluctuating or pulsatile light signal picked up by detector **310,** is referred to as a photoplethysmographic signal. Detector **310** converts this photoplethysmographic signal into electronic form and these photoplethysmographic data are then communicated back to the monitor **100** for processing into the data, analyte levels, hemodynamic parameters, and other information to be output by the display **120.**

As shown in FIG. 1, a plastic housing **340** provides the housing for the elements of the sensor **300** and is configured to clip onto the tissue-under-test **400.** In this example the tissue-under-test is a finger which is inserted into the sensor **300.** However, photoplethysmographic sensors **300** may come in a variety of configurations to be attached to various locations of the patient including fingers, ears, the nose, a forehead, a cheek, and, more recently, even on a wrist. These sensors may or may not be integrated with the patient cable **200.** The sensor **300** in this embodiment is meant to be representative of any type of photoplethysmographic sensor. Further, the sensor **300** can be transmissive, positioning the light source, in this case the output aperture **330,** and the detector **310** on opposite sides of the tissue-under-test such as is typically done in finger or ear sensors, or reflective, where the aperture **330** and detector **310** are positioned next to, or near to, each other on the same side of the tissue-under-test, as would be the case with a sensor **300** designed to be placed, for example, on the cheek or forehead. The sensor **300** can also be designed to be used in veterinary medicine and/or internally, for example in the esophagus, anus, external auditory meatus of the ear, or, during surgery, on an internal organ.

There are many potential embodiments of photoplethysmographic device(s) all of which are intended to be included within the scope of this invention. For example, in certain fingertip photoplethysmographic devices there is no separate sensor **300** or patient cable **200,** because, in those fingertip photoplethysmographic devices, the monitor **100,** sensor **300,** and patient cable **200,** are integrated into a single housing.

In yet another photoplethysmographic device embodiment, there may be no patient cable, and the laser housing **160** is included in the sensor **300** along with At least a portion of the analog signal processing **170** electronics. In this embodiment the sensor preferably communicates with the monitor **100** wirelessly.

The sensor may or may not include the photodetector or the light sources as these may be located remotely from the sensor and the light signals maybe transmitted, or communicated, to and/or from the senor by fiber optics. Herein, the term fiber optics, or optical fiber, is used to refer to a standard optical fiber, typically comprising a glass fiber with a core, cladding, and a buffer, a fiber bundle, or any other type of light pipe, or set of light pipes, glass, plastic, or otherwise, for conducting light.

Further the photoplethysmographic monitor and sensor may exist in a wide variety of forms. In a fingertip oximeter, for example, the photoplethysmographic monitor and sensor are integrated into a single unit including a display, an electronics module, and a sensor. By comparison the photoplethysmographic device can be broken out into a number of discrete components that, taken together still form the photoplethysmographic device. For example, in an operating room, the sensor might connect via a cable (or via radio frequency (RF) or other wireless communication modality) to an interface module, which may route the sensor signals to and from a separate electronics module, which may then send the output data to a central monitor, which then displays the photoplethysmographic measurements on one or more standalone displays.

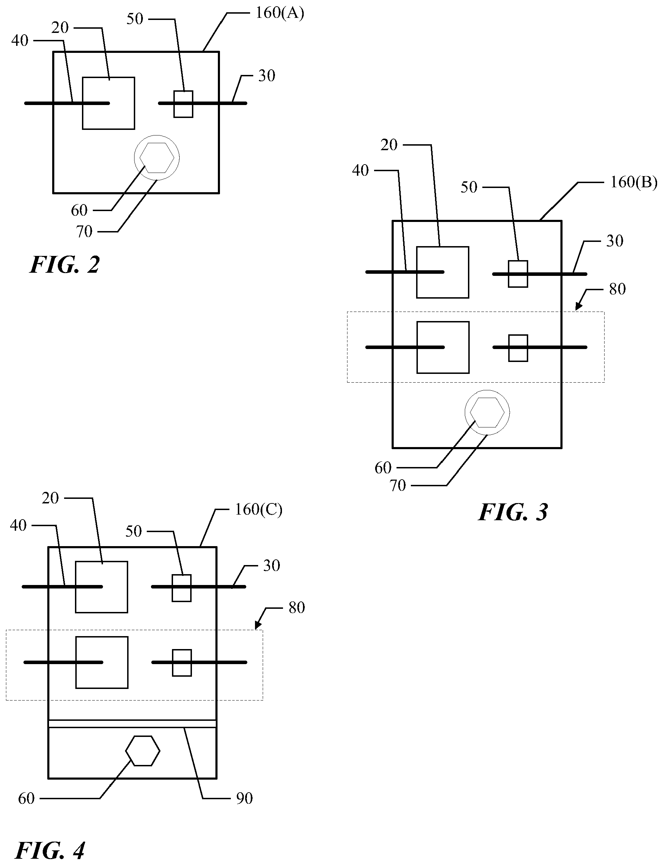

In the embodiment shown in FIG. 1 the cable **220** includes an optical fiber to communicate, or transmit, the diode laser light from the laser housing **160** to the sensor **300.** In a embodiment where the laser housing(s) **160** is positioned in the sensor there is no need for fiber optics in the cable **220.** Photoplethysmographic device **100** may also have an optical fiber **180** which conducts the light exiting the laser housing**160** to the monitor sensor connector **190.** The optical fiber **180** can be a continuation of the optical fiber(s) **30** (see FIG. 2) exiting the laser housing **160.** Alternatively, optical fiber **180** may be another configuration of light pipe, light pipes, or optical fiber(s) coupling the laser light out of the laser housing **160** to the monitor sensor connector **190.** In the embodiments where the laser housing**160** is positioned proximal to, or directly against, the tissue-under-test **400** optical fiber **180** can simply be replaced by an aperture to allow the light out of the fibers transiting the laser housing **160** to be directly (or possibly indirectly through additional optics such as a diffuser) incident on the tissue-under-test **400.** Thus, the light generated by the diode laser or lasers may be transmitted directly via a single optical fiber or through two or more serially-connected optical fibers from the diode laser **20** to the sensor and/or to the output aperture **330.**

FIG. 2 shows one embodiment of the laser housing **160.** In this embodiment the laser housing is a single laser housing **160(A)** that includes electrical connections **40** from the emitter drive electronics **150** to a diode laser **20.** Herein, the term 'electrical connections' is used to indicate any electrical conductors, such as wires, pins, or other elements, used to make electrical contact between different electrical elements in the photoplethysmographic device **100** such as the emitter drive electronics **150** and the diode laser **20.** Laser light emitted by the diode laser **20** is incident on, and coupled into, either directly or through additional optics, an optical fiber **30.** The optical fiber **30** is held in position within the laser housing **160(A)** by an optical mount **50** to maintain the optical coupling with the diode laser **20.** The optical mount **50** can be a drop of epoxy that holds the optical fiber **30** in place, a mechanical device that allows the optical fiber to be moved in position to the diode laser **20** to change or maximize the optical coupling efficiency with the diode laser **20,** or another device capable of maintaining the arrangement between the diode laser **20** and the optical fiber **30.**

While the diode laser **20** is fully enclosed within the laser housing **160(A),** the optical fiber **30** conducts light from the diode laser **20,** positioned inside the laser housing, to the outside of the laser housing **160(A).** Both the optical fiber **30** and the electrical connections **40** transit from inside the laser housing **160(A)** to the outside of the laser housing **410.** The optical fiber **30** connects to optical fiber **180,** becomes optical fiber **180,** or be connected directly to the sensor **300** to output the laser light directly toward the tissue-under-test **400.** Connections between the optical fiber **30** in the laser housing and the optical fiber **180,** or between optical fiber **180** and the optical fiber in the patient cable **200,** can be made through the use of industry-standard fiber coupling connectors such as, for example, SC, FC, LC, ST, or MT type connectors, or through the use of a custom fiber optical connector.

Optical fiber **180** may be a single optical fiber, or it may be comprised of multiple optical fibers coupled to the optical fibers **30** from multiple laser housings **160(A).** Alternatively, optical fiber **180** may be a group of optical fibers **30** from multiple laser housings **160(A)** and these fibers may terminate at the monitor sensor connector **190** where they can couple to the patient cable connector **210** when it is plugged into the photoplethysmographic monitor **100.**

Laser housing **160(A)** further encloses a desiccant **60** that adsorbs, or absorbs, moisture from its environment. The desiccant **60** can be made of any one of, or any combination of, a number of materials that will naturally adsorb, or absorb, moisture. These desiccant materials include, but are not limited to, silica gel, clay, activated charcoal, calcium sulfate, calcium chloride, calcium oxide, molecular sieve material, and zeolite.

The desiccant **60** enclosed within the laser housing **160(A)** is, in this embodiment, held in place by an adhesive **70** to prevent the desiccant **60** from making physical contact with the diode laser **20** or interfering with the optical path of the light exiting diode laser **20** and coupling into the optical fiber **30.** The adhesive used may be any one of a number of different adhesives, or combination of adhesives, including, but not limited to, epoxies, a silicone elastomer, a cyanoacrylate, and a hot melt glue. As shown in FIG. 2 adhesive **70** holds desiccant **60** outside of the optical path between the diode laser **20** and the optical fiber **30,** also keeping the desiccant **60** from shifting around inside the laser housing **160(A)** where it might damage the diode laser **20,** the delicate bond wires connecting electrical connections **40** to the diode laser **20,** or any other elements contained within the laser housing **160(A),** such as a back facet photodiode used to detect or monitor light emitted from the back facet of the diode laser **20.**

The adhesive **70** is preferably applied sparingly to minimize its impact on the water absorbing properties of the desiccant **60.** This may be accomplished by contacting only the amount of surface area on the desiccant 60 necessary to hold it in place within the laser housing**160(A).**

FIG. 3 shows another embodiment of a laser housing **160.** In this embodiment laser housing **160(B)** contains two or more diode lasers **20,** two or more sets of electrical connections **40,** two or more sets of optical mounts **50,** and two or more sets of optical fibers **30.** In this embodiment, once the laser housing **160(B)** is sealed, the desiccant **60** can adsorb, or absorb, the moisture from inside the laser housing **160(B),** protecting the two or more diode lasers **20** contained within the laser housing **160(B)** from moisture damage. The additional set, or sets, of elements is indicated in FIG. 3 by the dotted line box **80** which is intended to show an additional set of elements including, at least, the electrical connections **40,** the diode laser **20,** the optical mount **50,** and the optical fiber **30.** In this embodiment, all the optical fibers **30** and all electrical connections **40** must transit from inside to outside the sealed laser housing **160(B).**

Some of the advantages of this embodiment include the ability to use a single laser housing **160(B)** to house multiple diode lasers **20,** the possibility of reducing the number of electrical connections **40** that must transit the laser housing **160(B)** by, for example, using a common ground or power line or connector for multiple electrical elements within the laser housing **160(B),** and the ability to use a single desiccant **60** to protect multiple diode lasers **20.** The adhesive **70** prevents the desiccant from moving around inside the laser housing **160(B)** and potentially damaging one of the elements in the housing or interrupting the optical path of the one or more diode lasers **20.** It may also be desirable to combine the light out of multiple diode lasers **20** into a single optical fiber **30** within the laser housing **160(B)** to further reduce the number of items that have to transit from inside to outside the laser housing **160(B).** This further reduces the number of potential paths for moisture to migrate from outside to inside the laser housing **160(B)** by reducing the number of optical fibers **30** and or electrical connections **40** transiting the laser housing **160(B).**

FIG. 4 shows laser housing **160(C)** which is another embodiment of the laser housing **160.** This laser housing **160(C)** can contain one or more diode lasers **20** along with their associated optical fibers **30,** electrical connections **40,** and optical mounts **50.** An additional set, or sets, of elements is indicated in FIG. 4 by the dotted line box **80.** This embodiment of laser housing **430** is identical to laser housing **160(A)** or laser housing **160(B)** with the exception that the desiccant **60** is not held in place by an adhesive but is isolated from the rest of the inside of the laser housing **160(C)** by a physical separator **90.** The physical separator **90** allows movement of air between the portion of the laser housing **160(C)** containing the desiccant **60** and the rest of the laser housing containing the remaining elements. The physical separator **90,** as did the adhesive **70,** prevents the desiccant **60** from physically coming into contact with the other elements within the laser housing **160(C)** and may be used in place of the adhesive in a single diode laser housing configuration such as laser housing **410.** The physical separator **90** can be a wall separating the side of the laser housing **160(C)** containing the desiccant **60** from the side containing the other internal elements. It could also be a screen or another separator within the laser housing **160(C)** as long as the desiccant **60** is too large to fit through any opening from the desiccant **60** side to the side housing the one or more diode lasers **20.**

The desiccant **60** preferably adsorbs, or absorbs, moisture slowly. This can be achieved, for example, by selecting a desiccant **60** that, when exposed to ambient air for up to five minutes at 25°C, at 760mm of mercury atmospheric pressure, and at no more than 75% relative humidity, adsorbs or absorbs less than 25% of its moisture absorbing capacity. The laser housing **160** could then be fully manufactured under ambient air conditions but not sealed. The final steps in the manufacturing process would be to add the desiccant 60 into the housing and then to seal the housing.

The sealing process might be welding a lid onto the base of the laser housing **160,** or using an adhesive to attach the lid to create a seal. In either case, final sealing of the laser housing **160** could be accomplished under room air conditions as long as the desiccant **60** was removed from its dry environment, placed within the housing, and rapidly sealed so that the desiccant **60** only adsorbed a small percentage of the maximum amount of moisture that it is capable of adsorbing. By selecting a desiccant **60** that adsorbs moisture relatively slowly, the desiccant **60** could easily be exposed to typical room air environments for 5 minutes or more, during the sealing process, without adsorbing more than 25% of its capacity. This would guarantee at least 75% of the moisture absorbing capacity of the desiccant **60** was available after the laser housing was closed. The desiccant **60** would then have sufficient moisture absorbing capacity left to ensure that the relative humidity within the laser housing **160** would drop to an extremely low level, preferably to less than 3.5% at 25°C, at 760mm of mercury atmospheric pressure. Note that the construction of the laser housing **160** preferably provides a seal against the passage of moisture into the housing that is either hermetic or has a low enough water vaper transfer rate that the desiccant **60** can maintain a low internal humidity of the laser housing **160** as described herein, for an extended period of time. This time period may be months or years depending on the desired lifespan of the laser housing **160** once in use.

Dropping the internal relative humidity to such a low level would ensure that any moisture remaining in the laser housing **160** would not condense on the diode laser **20** even if the internal temperatures of the laser housing **160** dropped as low as -20°C, thus protecting the diode laser **20** and any other optical or electrical elements from potential moisture damage.

Another distinct advantage of this invention is that the laser housing **160** does not need to be fully hermetic. True hermetic packaging and sealing of electro-optical components, to prevent moisture from entering the housing, is difficult to accomplish and expensive, particularly for a housing with multiple internal diode lasers wherein multiple electrical connections and optical fibers must pass through the housing. This invention provides the ability to use an inexpensive, non-hermetic housing, with a low water-vapor-transfer-rate from outside the housing to inside the housing, and for the housing to be sealed under room air conditions, while still protecting the internal components from moisture damage. This allows for low-cost manufacturing, a lower cost housing, and a simpler manufacturing process.

Further, this invention allows the use of housing materials including aluminum that are known to retain moisture on the surfaces of the material. The moisture retention of aluminum means that even if an aluminum housing were sealed in a dry environment, the humidity inside the housing would immediately rise near to the humidity level outside the housing. This is true even if only part of the housing, for example the lid, is made of aluminum, or another material that reversibly holds moisture on its surface. The presence of a desiccant within the sealed housing ensures that as moisture is released from the housing material itself it is immediately adsorbed by the desiccant, thereby keeping the dew point within the housing low enough to prevent condensation even at temperatures down to -20°C.

The discussion of the embodiments has been presented for the purposes of illustration and description. The description is not intended to limit the invention to the form disclosed herein. Variations and modifications commensurate with the claims are considered to be within the scope of the present invention. The embodiments described herein are further intended to explain the best modes presently known of practicing the invention and to enable others skilled in the art to utilize the invention as such, or in other embodiments, and with the particular modifications required by their particular application or uses of the invention. It is intended that the appended claims be construed to include alternative embodiments to the extent permitted by the prior art.

Other embodiments and uses of the invention will be apparent to those skilled in the art from consideration of the specification and practice of the invention disclosed herein. All references cited herein, including all publications, U.S. and foreign patents and patent applications, are specifically and entirely incorporated by reference. It is intended that the specification and examples be considered exemplary only with the true scope and spirit of the invention indicated by the following claims. When used in this specification and claims, the terms "comprises" and "comprising" and variations thereof mean that the specified features, steps or integers are included. The terms are not to be interpreted to exclude the presence of other features, steps or components.

The invention may also broadly consist in the parts, elements, steps, examples and/or features referred to or indicated in the specification individually or collectively in any and all combinations of two or more said parts, elements, steps, examples and/or features. In particular, one or more features in any of the embodiments described herein may be combined with one or more features from any other embodiment(s) described herein.

Protection may be sought for any features disclosed in any one or more published documents referenced herein in combination with the present disclosure.

Although certain example embodiments of the invention have been described, the scope of the appended claims is not intended to be limited solely to these embodiments. The claims are to be construed literally, purposively, and/or to encompass equivalents.

### REPRESENTATIVE FEATURES

Representative features are set out in the following clauses, which stand alone or may be combined, in any combination, with one or more features disclosed in the text and/or drawings of the specification.
1. A photoplethysmographic device, comprising:
   a photoplethysmographic monitor;
   a laser housing electronically coupled to the photoplethysmographic monitor;
   at least two diode lasers, which generate laser light, positioned within the laser housing;
   the photoplethysmographic monitor providing electronic control of the at least two diode lasers;
   a desiccant fully enclosed within the laser housing;
   the desiccant positioned within the laser housing such that the desiccant does not make physical contact with the at least two diode lasers and/or interfere with the laser light; and
   a sensor in communication with the photoplethysmographic monitor and adapted to output the laser light from the at least two diode lasers;
   the sensor comprising a photodetector, wherein the photoplethysmographic monitor receives and processes photoplethysmographic data from the sensor.
2. The device of clause 1, wherein the desiccant is held in position within the laser housing by an adhesive.
3. The device of clause 2, wherein the adhesive is at least one of an epoxy, a silicone elastomer, a cyanoacrylate, a hot melt glue, and combinations thereof.
4. The device of any one of the preceding clauses, wherein the desiccant is isolated from the at least two diode lasers by a physical separator positioned within the laser housing, wherein the physical separator allows air movement between the desiccant and the at least two diode lasers.
5. The device any one of the preceding clauses, wherein the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity.
6. The device of any one of the preceding clauses, wherein the laser housing further comprises at least one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor.
7. The device of any one of the preceding clauses, wherein the laser housing is non-hermetic.
8. The device of any one of the preceding clauses, wherein the laser housing includes at least one aluminum part.
9. The device of any one of the preceding clauses, wherein the desiccant is at least one of silica gel, clay, activated charcoal, calcium sulfate, calcium chloride, calcium oxide, molecular sieve material, zeolite, and combinations thereof.
10. The device of any one of the preceding clauses, wherein the desiccant drops the relative humidity within the laser housing to less than 3.5% at 25°C, 760mm of mercury pressure.
11. A photoplethysmographic device, comprising:
   a photoplethysmographic monitor;
   at least two laser housings electronically coupled to the photoplethysmographic monitor;
   at least one diode laser, which generates laser light, positioned within each of the at least two laser housings;
   the photoplethysmographic monitor providing electronic control of each of the diode lasers;
   a desiccant fully enclosed within each of the at least two laser housings;
   each desiccant positioned within each of the at least two laser housings such that each desiccant does not make physical contact with the diode lasers and/or interfere with the laser light; and
   a sensor in communication with the photoplethysmographic monitor and adapted to output the laser light from the diode lasers;
   the sensor comprising a photodetector, wherein the photoplethysmographic monitor receives and processes photoplethysmographic data from the sensor.
12. The device of clause 11, wherein the desiccant is held in position within the laser housing by an adhesive.
13. The device of clause 11 or 12, wherein the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity.
14. The device of any one of clauses 11 to 13, wherein each laser housing further comprises one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor.
15. The device of any one of clauses 11 to 14, wherein each laser housing is non-hermetic.
16. The device of any one of clauses 11 to 15, wherein the desiccant is at least one of silica gel, clay, activated charcoal, calcium sulfate, calcium chloride, calcium oxide, molecular sieve material, zeolite, and combinations thereof.
17. The device of any one of clauses 11 to 16, wherein the desiccant drops the relative humidity within the laser housing to less than 3.5% at 25°C, 760mm of mercury pressure.
18. A method of manufacturing a photoplethysmographic device, comprising the steps of:
   providing a photoplethysmographic monitor;
   providing at least two diode lasers that each generate laser light;
   positioning each diode laser within a laser housing, wherein one or more diode lasers are contained within one or more laser housings;
   electronically coupling each diode laser to the photoplethysmographic monitor;
   positioning a desiccant within each of the one or more laser housings, wherein the desiccant is positioned such that the desiccant does not interfere with the laser light; and
   coupling a sensor to the photoplethysmographic monitor, wherein the sensor is in communication with the photoplethysmographic monitor and is adapted to guide the laser light from the diode lasers into a tissue-under-test, and wherein the photoplethysmographic monitor receives and processes photoplethysmographic data from the sensor.
19. The method of clause 18, wherein the step of positioning the desiccant within each laser housing is by an adhesive.
20. The method of clause 19, wherein the adhesive is at least one of an epoxy, a silicone elastomer, a cyanoacrylate, a hot melt glue, and combinations thereof.
21. The method of any one of clauses 18 to 20, wherein the desiccant is selected to adsorb less than 25% of its maximum water absorption capacity when exposed for less than 5 minutes to ambient air at standard temperature and pressure and no more than 75% relative humidity, whereby the desiccant can be sealed within the laser housing in ambient air while still maintaining at least 75% of the desiccant's water absorption capacity.
22. The method of any one of clauses 18 to 21, further comprising coupling one optical fiber to each diode laser, the optical fiber transiting the associated housing and transmitting the laser light emitted by the diode laser to the sensor directly or through two or more serially-connected optical fibers or other light pipes.
23. The method of any one of clauses 18 to 22, wherein the sensor comprises a laser light output aperture and a photodetector and wherein the sensor is capable of transmitting photoplethysmographic data from the photodetector to the photoplethysmographic monitor.
24. The method of any one of clauses 18 to 23, wherein at least one of the one or more laser housings is non-hermetic.
25. The method of any one of clauses 18 to 24, wherein the desiccant is at least one of silica gel, clay, activated charcoal, calcium sulfate, calcium chloride, calcium oxide, molecular sieve material, zeolite, and combinations thereof.
26. The method of any one of clauses 18 to 25, wherein the desiccant reduces the relative humidity within the at least one or more laser housings to less than 3.5% at 25°C, 760mm of mercury pressure.

## Claims

1. A photoplethysmographic device, comprising:
a photoplethysmographic monitor;
a laser housing electronically coupled to the photoplethysmographic monitor;
at least two diode lasers, which generate laser light, positioned within the laser housing;
the photoplethysmographic monitor providing electronic control of the at least two diode lasers;
a desiccant fully enclosed within the laser housing;
the desiccant positioned within the laser housing such that the desiccant does not make physical contact with the at least two diode lasers and/or interfere with the laser light; and
a sensor in communication with the photoplethysmographic monitor and adapted to output the laser light from the at least two diode lasers;
the sensor comprising a photodetector, wherein the photoplethysmographic monitor receives and processes photoplethysmographic data from the sensor.

2. The device of claim 1, wherein the desiccant is held in position within the laser housing by an adhesive.

3. The device of claim 2, wherein the adhesive is at least one of an epoxy, a silicone elastomer, a cyanoacrylate, a hot melt glue, and combinations thereof.

4. The device of any one of the preceding claims, wherein the desiccant is isolated from the at least two diode lasers by a physical separator positioned within the laser housing, wherein the physical separator allows air movement between the desiccant and the at least two diode lasers.

5. The device of any one of the preceding claims, wherein the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity.

6. The device of any one of the preceding claims, wherein the laser housing further comprises at least one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor.

7. The device of any one of the preceding claims, wherein the laser housing is non-hermetic.

8. The device of any one of the preceding claims, wherein the laser housing includes at least one aluminum part.

9. The device of any one of the preceding claims, wherein the desiccant is at least one of silica gel, clay, activated charcoal, calcium sulfate, calcium chloride, calcium oxide, molecular sieve material, zeolite, and combinations thereof.

10. The device of any one of the preceding claims, wherein the desiccant drops the relative humidity within the laser housing to less than 3.5% at 25°C, 760mm of mercury pressure.

11. A photoplethysmographic device, comprising:
a photoplethysmographic monitor;
at least two laser housings electronically coupled to the photoplethysmographic monitor;
at least one diode laser, which generates laser light, positioned within each of the at least two laser housings;
the photoplethysmographic monitor providing electronic control of each of the diode lasers;
a desiccant fully enclosed within each of the at least two laser housings;
each desiccant positioned within each of the at least two laser housings such that each desiccant does not make physical contact with the diode lasers and/or interfere with the laser light; and
a sensor in communication with the photoplethysmographic monitor and adapted to output the laser light from the diode lasers;
the sensor comprising a photodetector, wherein the photoplethysmographic monitor receives and processes photoplethysmographic data from the sensor.

12. The device of claim 11, wherein the desiccant is held in position within the laser housing by an adhesive, and optionally
wherein the desiccant adsorbs less than 25% of its maximum water absorption capacity when exposed for less than 5 minutes to ambient air at 25°C, 760mm of mercury pressure, and at no more than 75% relative humidity, whereby the desiccant can be rapidly sealed within the laser housing while in an ambient air environment while still maintaining water absorption capacity, and optionally,
wherein each laser housing further comprises one optical fiber coupled to each diode laser, each optical fiber transiting the laser housing, whereby the optical fibers transiting the laser housing or a set of one or more optical fibers coupled to the optical fibers transiting the laser housing transmit the laser light to the sensor, and optionally wherein each laser housing is non-hermetic, and optionally
wherein the desiccant is at least one of silica gel, clay, activated charcoal, calcium sulfate, calcium chloride, calcium oxide, molecular sieve material, zeolite, and combinations thereof, and optionally
wherein the desiccant drops the relative humidity within the laser housing to less than 3.5% at 25°C, 760mm of mercury pressure.

13. A method of manufacturing a photoplethysmographic device, comprising the steps of:
providing a photoplethysmographic monitor;
providing at least two diode lasers that each generate laser light;
positioning each diode laser within a laser housing, wherein one or more diode lasers are contained within one or more laser housings;
electronically coupling each diode laser to the photoplethysmographic monitor;
positioning a desiccant within each of the one or more laser housings, wherein the desiccant is positioned such that the desiccant does not interfere with the laser light; and
coupling a sensor to the photoplethysmographic monitor, wherein the sensor is in communication with the photoplethysmographic monitor and is adapted to guide the laser light from the diode lasers into a tissue-under-test, and wherein the photoplethysmographic monitor receives and processes photoplethysmographic data from the sensor.

14. The method of claim 13, wherein the step of positioning the desiccant within each laser housing is by an adhesive, and optionally wherein the adhesive is at least one of an epoxy, a silicone elastomer, a cyanoacrylate, a hot melt glue, and combinations thereof.

15. The method of claim 13 or 14, wherein the desiccant is selected to adsorb less than 25% of its maximum water absorption capacity when exposed for less than 5 minutes to ambient air at standard temperature and pressure and no more than 75% relative humidity, whereby the desiccant can be sealed within the laser housing in ambient air while still maintaining at least 75% of the desiccant's water absorption capacity, and optionally
further comprising coupling one optical fiber to each diode laser, the optical fiber transiting the associated housing and transmitting the laser light emitted by the diode laser to the sensor directly or through two or more serially-connected optical fibers or other light pipes and optionally
wherein the sensor comprises a laser light output aperture and a photodetector and wherein the sensor is capable of transmitting photoplethysmographic data from the photodetector to the photoplethysmographic monitor, and optionally
wherein at least one of the one or more laser housings is non-hermetic, and optionally wherein the desiccant is at least one of silica gel, clay, activated charcoal, calcium sulfate, calcium chloride, calcium oxide, molecular sieve material, zeolite, and combinations thereof, and optionally
wherein the desiccant reduces the relative humidity within the at least one or more laser housings to less than 3.5% at 25°C, 760mm of mercury pressure.
